(19) Europäisches Patentamt
European Patent Office
Office européen des brevets

(11) Publication number: 0 256 472 A2

(12) **EUROPEAN PATENT APPLICATION**

(21) Application number: 87111541.6

(51) Int. Cl.$^4$: A61K 7/48

(22) Date of filing: 10.08.87

(30) Priority: 11.08.86 JP 187983/86
18.09.86 JP 220212/86

(43) Date of publication of application:
24.02.88 Bulletin 88/08

(84) Designated Contracting States:
AT BE CH DE ES FR GB IT LI NL SE

(71) Applicant: LION CORPORATION
3-7, Honjo 1-chome
Sumida-ku Tokyo(JP)

(72) Inventor: Sugiyama, Keikichi
18-13, Ishigamidai 1-chome Oiso-machi
Naka-gun Kanagawa-ken(JP)
Inventor: Takada, Koji
909-2, Daigiri
Fujisawa-shi Kanagawa-ken(JP)
Inventor: Yamamoto, Ikuo
Copo-Gurasu 402 585-24, Renshoji
Odawara-shi Kanagawa-ken(JP)

(74) Representative: Kuhnen, Wacker & Partner
Schneggstrasse 3-5 Postfach 1729
D-8050 Freising(DE)

(54) **Cosmetic agent for application to skin.**

(57) A cosmetic agent for application to skin such as facial cream comprises an essential compound selected from the group consisting of 5'-deoxyadenosylcobalamine, flavin adenine dinucleotide, β-nicotinamide adenine dinucleotide, β-nicotinamide adenine dinucleotide phosphate, coenzyme A, pyrrolo-quinoline quinone and guanosine 3',5'-cyclic monophosphate or derivatives thereof.

The cosmetic agent has the effects of activating dermal cells and promoting metabolism, and therefore the cosmetic agent is useful for preventing aging of the skin and providing wrinkle-free, smooth, moist and young-looking skin.

EP 0 256 472 A2

Xerox Copy Centre

# Cosmetic Agent for Application to Skin

## BACKGROUND OF THE INVENTION

### Field of the Invention

The present invention relates to agents for application to the skin, such as facial creams, which have the effects of activating dermal cells and promoting metabolism and which are used for preventing aging of the skin and providing wrinkle-free, smooth, moist, and young-looking skin.

### Prior Art

Although animal and plant extracts, vitamins, amino acids, and nucleic acids which are extracted from natural substances have been conventionally mixed into cosmetics for the purpose of preventing aging of the skin, no marked effect concerning the prevention of aging can be expected, even if a relatively large amount of these is mixed in, because they have only auxiliary functions.

It has also been proposed that prostaglandins which are physiologically active substances present in small amounts in organisms (Japanese Patent Un-Examined Publication (KOKAI) No.18436/1973), EGF (epidermal growth factor), or urogastrone could be utilized in cosmetics (Japanese Patent Un-Examined Publication (KOKAI) No.5006/1986). These methods, however, are of little practical use for the following reasons:

(i) Since these compounds are substances having high molecular weights, they are not able to penetrate skin cells easily and thus cannot exhibit sufficient effect.

(ii) Since EGF and urogastrone are proteins, they are easily decomposed by the proteases in cells and thus cannot exhibit sufficient effect.

(iii) In addition, there are problems with respect to safety such as danger of side-effects.

## SUMMARY OF THE INVENTION

It is a primary object of the present invention to provide cosmetic agents for application to the skin which exhibit excellent effects concerning the prevention of aging of the skin and which have no side-effects, are extremely safe, and are practicable.

This and other objects of the present invention will be clear from the following description.

The present invention has been achieved on the basis of the knowledge that the above-described problems can be effectively solved by mixing into a cosmetic agent for application to the skin some specific coenzyems which are present in small amounts in various tissues and cells of organisms and serve as the essential components of various enzyme reactions supporting the metabolism of various substances, or specific nucleic acid related substances which are thought to have various regulatory functions.

In accordance with the present invention there are provided cosmetic agents for application to the skin, comprising at least one compound selected from the group consisting of 5'-deoxyadenosylcobalamine or salts thereof, flavin adenine dinucleotide or salts thereof; β-nicotinamide adenine dinucleotide, a reduced form thereof, or their salts; β-nicotinamide adenine dinucleotide phosphate, a reduced form thereof, or their salts; coenzyme A or salts thereof; pyrroloquinoline quinone or salts thereof; and compounds having a fundamental skeleton given by the following formula:

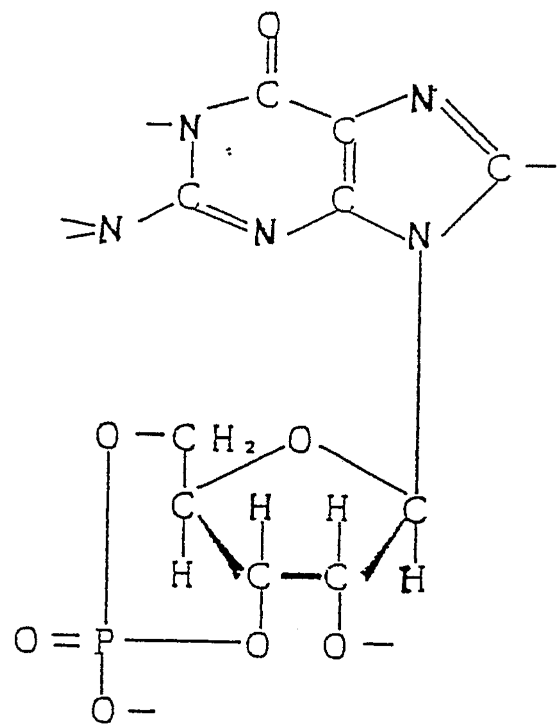

... [ I ]

DESCRIPTION OF THE PREFERRED EMBODIMENTS

5'-deoxyadenosylcobalamine used in the present invention is called coenzyme $B_{12}$ (referred to as $CoB_{12}$ hereinafter) and is a substance differing from cyanocobalamine, hydroxocobalamine, and methylcobalamine which are generally used as vitamins $B_{12}$ in pharmaceuticals or cosmetics. It is known that $CoB_{12}$ is widely present in very small amounts in biological tissues and cells and is an essential component as a coenzyme for enzymes which are important in the metabolism of substances, such as methylmalonyl-CoA mutase. Although $CoB_{12}$ is available only as a biochemical reagent on the market at the present, it can be produced from vitamin $B_{12}$ (cyanocobalamine) by an organic chemical method or biochemical method ("Vitamins and Coenzymes" in Seikagaku Jikken Koza, Vol. 13, the last volume, pp455 - 458).

Salts of $CoB_{12}$ can also be used in the present invention and examples of such salts include $CoB_{12}$ sodium salt ($CoB_{12}$ Na) and $CoB_{12}$ potassium salt ($CoB_{12}$ K).

Flavin adenine dinucleotide (referred to as FAD hereinafter) is known as an important substance which takes part as a coenzyme of oxidoreductase in the general oxidation of organic compounds in an organism. In addition, FAD is widely used as a pharmaceutical material for the relief of dermatitis, allergic diseases, stomatitis, and beriberi, and as an active ingredient of eye lotions.

Currently, FAD is industrially produced by fermentation or synthesis methods and can easily be obtained. Salts of FAD can also be used in the present invention, and examples of such salts include FAD sodium salt (FAD 2Na) and FAD potassium salt (FAD 2K).

$\beta$-nicotinamide adenine dinucleotide (referred to as NAD hereinafter) and $\beta$-nicotinamide adenine dinucleotide phosphate (referred to as NADP hereinafter) are known as important substances which serve as coenzymes of various dehydrogenases in redox reactions in organisms. These coenzymes are currently used as diagnostic medicines and biochemical reagents, and coenzymes mainly produced by fermentation methods are available on the market. Reduced forms of NAD and NADP (referred to as NADH and NADPH, respectively) can also be used in the present invention. Examples of such substances which can be used in the present invention include NAD sodium salt (NAD Na), NAD lithium salt (NAD Li), NAD potassium salt (NAD K), NADP sodium salt (NADP Na), NADP potassium salt (NADP K), NADP tris-salt, NADH sodium salt (NADH 2Na), NADH potassium salt (NADH 2K), NADH cyclohexylamine salt, NADH tris-salt, NADPH sodium salt (NADPH Na), NADPH potassium salt (NADPH K) NADPH cyclohexylamine salt, and NADPH tris-salt.

A coenzyme A (referred to as CoA hereinafter) takes part in the transfer reaction of an acyl group in an organism, and is known as an important substance which serves as a carrier for acyl groups in various enzyme reactions such as the oxidation of fatty acids, the synthesis of fatty acids, the synthesis of steroids, the oxidation of pyruvic acid, the metabolism of amino acids and biological acetylation. CoA is used as a pharmaceutical material in Europe and CoA produced by fermentation methods is available on the market in Japan in the form of diagnostic medicines and reagents. Salts of CoA can also be used in the present invention, and examples of such salts include CoA sodium salts (CoA Na, CoA 3Na), CoA lithium salts (CoA Li, CoA 3Li), and CoA potassium salts (CoA K, CoA 3K).

Pyrrolo-quinoline quinone (referred to as PQQ hereinafter) has been vigorously studied and has been known to be widely distributed in organisms since it was discovered in 1979 as a coenzyme of methanol dehydrogenase with respect to bacteria which oxidize methanol. Although it has been reported that PQQ has a coenzyme function for dehydrogenase or oxidase and a promotion function of cell growth (Japanese Patent Un-Examined Publication (KOKAI) No.58584/1986), there has been no report on any other physiological functions of PQQ.

PQQ produced by fermentation using bacteria which oxidize methanol, is currently available as a biochemical reagent on the market. Salts of PQQ can also be used in the present invention and examples of such salts include PQQ potassium salt (PQQ 2K) and PQQ sodium salt (PQQ 2Na).

In addition, any compounds having the fundamental skeleton expressed by Formula [I] can be used in the present invention. Preferably, guanosine 3',5'-cyclic monophosphate (referred to as cGMP hereinafter) expressed by the Formula below (where $R_1$, $R_2$, $R_3$, $R_4$, X and M are each a hydrogen compound), specific derivatives thereof, or their salts can be used.

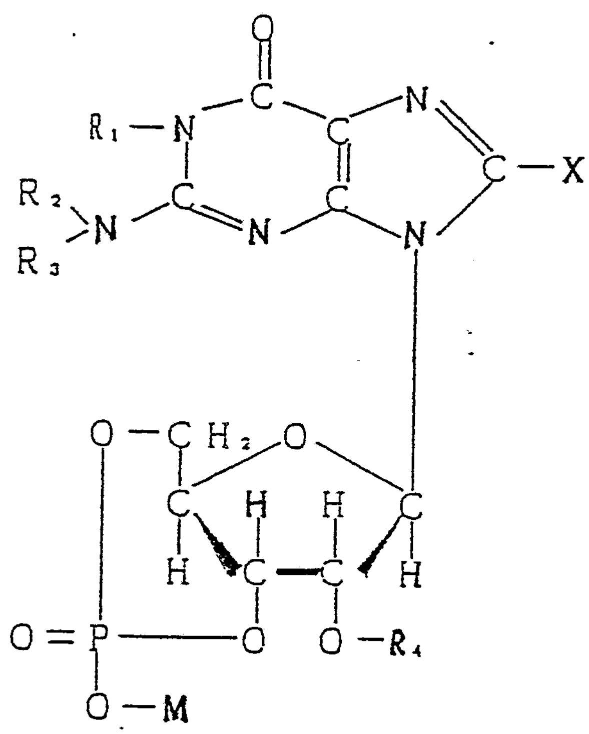

··· [II]

(wherein $R_1$, $R_2$, $R_3$, and $R_4$ are each a hydrogen atom, an acyl group having 1 to 22 carbon atoms, or an alkyl group having 1 to 22 carbon atoms; X is a hydrogen atom, a halogen atom, a mercapto group, a thio-containing group, an amino group, an aminoalkyl group, or a hydroxyl group; and M is a hydrogen atom or a salt-forming cation).

It is preferable that each of $R_1$, $R_2$, $R_3$, and $R_4$ in Formula [II] be a hydrogen atom; an acyl group having 1 to 22 carbon atoms, more particularly 1 to 12 carbon atoms; or an alkyl group having 1 to 22 carbon atoms, more particularly 1 to 12 carbon atoms.

In addition, X is preferably a hydrogen atom, a halogen atom such as a bromine, iodine, chlorine, or fluorine atom, a mercapto group, a thio-containing group such as a thioalkyl group having 1 to 4 carbon atoms, a 4-chlorophenylthio group, or a thiobenzyl group, an amino group, an aminoalkyl group having 1 to 4 carbon atoms, or a hydroxyl group; and M is preferably a hydrogen atom, an atom of an alkali metal such as sodium, potassium, or lithium, or tris(hydroxymethyl)aminomethane.

An acyl group and an alkyl group may each have a substituent such as a halogen atom or an aromatic ring, and an acyl group may be originated from a dibasic acid.

cGMP used in the present invention is present in a very small amount in organisms and has recently been recognized to have importance in that it has regulatory functions with respect to various hormone actions. At present, cGMP and various derivatives thereof which are produced by fermentation or synthetic methods are available as biochemical reagents on the market.

Examples of compounds expressed by Formula [II] are given in Table 1, and mixtures of one or more of these compounds and sodium salts, potassium salts, or lithium salts thereof are used in the present invention.

In Table 1, ClPhS represents a 4-chlorophenylthio group.

Table 1

| Group | $R_1$ | $R_2$ | $R_3$ | $R_4$ | X |
|---|---|---|---|---|---|
| 1 | H | H | H | H | H |
| 2 | Acetyl | H | H | H | H |
| | Butyryl | H | H | H | H |
| | Hexanoyl | H | H | H | H |
| | Succinyl | H | H | H | H |
| | Ethyl | H | H | H | H |
| 3 | Acetyl | H | H | H | Cl |
| | Butyryl | H | H | H | Br |
| | Succinyl | H | H | H | ClPhS |
| 4 | H | Acetyl | H | H | H |
| | H | Butyryl | H | H | H |
| | H | Hexanoyl | H | H | H |
| | H | Octanoyl | H | H | H |
| | H | Lauroyl | H | H | H |
| | H | Malonyl | H | H | H |
| | H | Succinyl | H | H | H |
| | H | Benzoyl | H | H | H |
| | H | Methyl | H | H | H |
| | H | Ethyl | H | H | H |
| | H | Ethyl | Ethyl | H | H |

| Group | $R_1$ | $R_2$ | $R_3$ | $R_4$ | X |
|---|---|---|---|---|---|
| 5 | H | Acetyl | H | H | Amino |
| | H | Acetyl | H | H | Aminomethyl |
| | H | Butyryl | H | H | Br |
| | H | Butyryl | H | H | ClPhS |
| | H | Succinyl | H | H | Br |
| | H | Succinyl | H | H | ClPhS |
| | H | Ethyl | H | H | Cl |
| | H | Ethyl | H | H | Mercapto |
| 6 | H | H | H | Acetyl | H |
| | H | H | H | Butyryl | H |
| | H | H | H | Hexanoyl | H |
| | H | H | H | Octanoyl | H |
| | H | H | H | Lauroyl | H |
| | H | H | H | Malonyl | H |
| | H | H | H | Succinyl | H |
| | H | H | H | Benzoyl | H |
| | H | H | H | Methyl | H |
| | H | H | H | Ethyl | H |

| Group | $R_1$ | $R_2$ | $R_3$ | $R_4$ | X |
|---|---|---|---|---|---|
| 7 | H | H | H | Acetyl | Amino |
| | H | H | H | Acetyl | Aminomethyl |
| | H | H | H | Butyryl | Br |
| | H | H | H | Butyryl | ClPhS |
| | H | H | H | Succinyl | Br |
| | H | H | H | Succinyl | ClPhS |
| | H | H | H | Ethyl | Cl |
| | H | H | H | Ethyl | Mercapto |
| 8 | H | H | H | H | Br |
| | H | H | H | H | Cl |
| | H | H | H | H | Mercapto |
| | H | H | H | H | Thiomethyl |
| | H | H | H | H | ClPhS |
| | H | H | H | H | Amino |
| | H | H | H | H | Aminomethyl |
| | H | H | H | H | Hydroxyl |
| 9 | Acetyl | Acetyl | H | H | H |
| | Butyryl | Butyryl | H | H | H |
| | Succinyl | Succinyl | H | H | H |
| 10 | Acetyl | Acetyl | H | H | Cl |
| | Butyryl | Butyryl | H | H | Br |
| | Succinyl | Succinyl | H | H | ClPhS |

| Group | $R_1$ | $R_2$ | $R_3$ | $R_4$ | X |
|---|---|---|---|---|---|
| 11 | H | Acetyl | H | Acetyl | H |
| | H | Butyryl | H | Butyryl | H |
| | H | Hexanoyl | H | Hexanoyl | H |
| | H | Octanoyl | H | Octanoyl | H |
| | H | Lauroyl | H | Lauroyl | H |
| | H | Malonyl | H | Malonyl | H |
| | H | Succinyl | H | Succinyl | H |
| | H | Benzoyl | H | Benzoyl | H |
| | H | Methyl | H | Methyl | H |
| | H | Ethyl | H | Ethyl | H |
| 12 | H | Acetyl | H | Acetyl | Amino |
| | H | Acetyl | H | Acetyl | Aminomethyl |
| | H | Butyryl | H | Butyryl | Br |
| | H | Butyryl | H | Butyryl | ClPhS |
| | H | Succinyl | H | Succinyl | Br |
| | H | Succinyl | H | Succinyl | ClPhS |
| | H | Ethyl | H | Ethyl | Cl |
| | H | Ethyl | H | Ethyl | Mercapto |
| 13 | Acetyl | H | H | Acetyl | H |
| | Butyryl | H | H | Butyryl | H |
| | Succinyl | H | H | Succinyl | H |

| Group | $R_1$ | $R_2$ | $R_3$ | $R_4$ | X |
|---|---|---|---|---|---|
| 14 | Acetyl<br>Butyryl<br>Succinyl | H<br>H<br>H | H<br>H<br>H | Acetyl<br>Butyryl<br>Succinyl | Cl<br>Br<br>ClPhS |
| 15 | Acetyl<br>Butyryl<br>Succinyl | Acetyl<br>Butyryl<br>Succinyl | H<br>H<br>H | Acetyl<br>Butyryl<br>Succinyl | H<br>H<br>H |
| 16 | Acetyl<br>Butyryl<br>Succinyl | Acetyl<br>Butyryl<br>Succinyl | H<br>H<br>H | Acetyl<br>Butyryl<br>Succinyl | Cl<br>Br<br>ClPhS |

The above-described coenzymes and nucleic acid related substances which are used in the present invention are generally contained in amounts of 0.001 to 5% by weight (referred to as % hereinafter), preferably 0.01 to 2%, in various types of cosmetic agents for application to the skin.

In addition to the above-described essential components, other materials which are ordinarily used in cosmetic agents for application to the skin, can be mixed into the cosmetic agent for application to the skin of the present invention. For example, oil, water, surfactants, humectants, lower alcohols, thickening agents, antioxidants, chelating agents, pH-adjustors, preservatives, perfumes, or color additives can be mixed into the agent. Examples of oils include fats and oils such as olive oil, jojoba oil, and hardened oil; waxes such as spermaceti, beeswax, and lanolin; hydrocarbons such as liquid paraffin, ceresin, and squalane; fatty acids such as stearic acid and oleic acid; alcohols such as cetanol, stearyl alcohol, lanolin alcohol, and hexyl decanol; and esters such as isopropyl myristate and butyl stearate. Examples of surfactants include anionic surfactants such as sodium stearate, sodium cetylsulfate, polyoxyethylene laurylether phosphate, sodium N-acyl glutamate; cationic surfactants such as stearyldimethylbenzylammonium chloride and stearyltrimethylammonium chloride; ampholytic surfactants such as alkylaminoethylglycine hydrochloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, polyoxyethylene polyoxypropylene glycol, polyoxyethylene castor oil, and polyoxyethylene lanolin. Examples of humectants include glycerin, 1,3-butylene glycol, and propylene glycol; examples of lower alcohols include ethanol and isopropanol; examples of thickening agents include polyethylene glycol and sodium carboxymethylcellulose; examples of antioxidants include dibutylhydroxytoluene, butylhydroxyanisole, and propyl gallate; examples of chelating agents include disodium edetate and ethanehydroxy diphosphate; examples of pH adjustors include citric acid, sodium citrate, boric acid, borax, and disodium hydrogen phosphate; and examples of preservatives include methyl para hydroxybenzoate, ethyl para-hydroxybenzoate, dehydroacetic acid, salicylic acid, and benzoic acid. Optional components are not limited to these compounds. Appropriate mixing of the above-described essential components and optional components enables them to be used in various product forms such as toilet waters, creams, packs, lotions, skin milks, or milky lotions. An example of a cosmetic agent for application to skin is a composition containing 0.001 to 5% of the essential components of the present invention, 0.5 to 12% of a surfactant, 2 to 15% of a humectant, 0 to 80% of an oil, very small amount of preservative and 11 to 95% of purified water.

An example of a toilet water is a composition containing 0.01 to 2% of the essential components of the present invention, 2 to 10% of a lower alcohol, 0.5 to 1% of a surfactant, 3 to 7% of a humectant, 0.05 to 0.2% of a pH adjustor, 80 to 95% of purified water, and very small amounts of preservative, color additive, and perfume.

An example of a skin cream is a composition containing 0.01 to 2% of the essential components, 20 to 70% of oil, 2 to 7% of a surfactant, 5 to 10% of a humectant, 11 to 73% of purified water, and very small amounts of preservative and perfume.

An example of a lotion is a composition containing 0.01 to 2% of the essential components, 5 to 10% of a lower alcohol, 0.5 to 2% of a surfactant, 2 to 8% of a humectant, 0.01 to 0.05% of an antioxidant, 0.02 to 0.1% of a chelating agent, 0.1 to 1% of a pH adjustor, 77 to 92% of purified water, and very small amounts of preservative and perfume.

An example of a skin milk is a composition containing 0.01 to 2% of the essential components, 20 to 40% of oil, 2 to 5% of a surfactant, 53 to 78% of purified water, and very small amounts of preservative and perfume.

An example of a milky lotion is a composition containing 0.01 to 2% of the essential components, 10 to 30% of oil, 1 to 5% of a surfactant, 5 to 10% of a humectant, 53 to 84% of purified water, and very small amounts of preservative and perfume.

Although it is not known in detail how the cosmetics of the present invention exhibit an excellent function of preventing the aging of the skin, it is assumed that a major cause for the aging of the skin is based on the decrease of an amount of fibril proteins such as collagen and elastin constituting the derma, the modification of the structure thereof and weakening thereof, rather than the modification of the epidermis on the surface of the skin. Therefore, as a result of broad study based on the later mentioned test samples to find out the chemical compounds of promoting the growth of dermal fibroblasts which produce and secrete collagen and elastin in the derma, it was made clear that the essential components of the present invention have excellent function of promoting the production of collagen and its fibrillation and also it was confirmed that the cosmetics of the present invention have an excellent function of preventing the aging of the skin to humans, as shown in the examples. Therefore, when the cosmetics of the present invention are applied to the skin, the cosmetics exhibit the above-described excellent function as a result of the action of promoting the growth of dermal fibroblasts, the action of promoting the production of collagen elastin fibrils and the action of promoting the respiratory ability of dermal cells, as well as the action of activating the TCA cycle of dermal cells, which actions are caused by endermic absorption.

The above-described coenzymes and nucleic acid related substances used in the present invention are widely present in organisms and are thus thought to be extremely safe. When the level of safety was checked in order to make sure, no substantial problem was observed with respect to acute toxicity or skin irritation, skin sensitization, and a high level of safety was recognized.

In accordance with the present invention, it is possible to provide cosmetic agents for application to the skin which can prevent the occurrence of wrinkles and produce smooth, moist, young-looking skin, and which exhibit a remarkably excellent effect as compared with conventional agents, as well as a very high level of safety.

Therefore, the cosmetic agents for application to the skin of the present invention can be widely used in various forms such as various kinds of cosmetic creams, toilet waters, lotions, skin milks, milky lotions and packs.

The present invention will now be described in detail with reference to tests and examples.

Test Example

Test Example 1

The action of promoting the growth of dermal fibroblasts by the coenzymes and nucleic acid related substances used in the present invention was evaluated in accordance with the following method:

Fibroblasts originating in the human skin were cultured in media to which the above-described compounds had been added and to which no such compound was added under serum controlled conditions and the effect of the compounds on proliferation was examined. That is, the above-described fibroblasts were cultured in a $Co_2$-incubator at 37°C for 24 hours in Eagle's MEM medium (pH 7.6) to which were added 10% of fetal bovine serum and the like. The medium was then changed to a medium in which the serum was lowered 1% and in which the fibroblasts were then cultured for 24 hours. The medium was then changed into a medium containing $10^{-5}$ M of a substance for examination and 1% of serum in which the culture was continued.

The medium was then changed on alternate days. The results obtained are shown in Table 2 in which the number of cells for each case is shown by a relative value obtained by assuming the number of cells after 14 days to be 100 in the case of the control in which no substance for examination was added.

The abbreviations given below are used in Table 2.
MB: monobutyryl
MS: monosuccinyl
ClPhS: 4-chlorophenylthio
DB: dibutyryl
DS: disuccinyl

In addition, the position of the substituents in cGMP derivatives is shown as follows:
$N^2$: substituted in $R_2$ or $R_3$
$O^{2'}$: substituted in R4
8: substituted in X

Table 2

| Test substance | Number of cells |
|---|---|
| No substance added (control) | 100 |
| $CoB_{12}$ | 179 |
| FAD 2Na | 147 |
| NAD | 154 |
| NADH 2Na | 151 |
| NADP | 141 |
| NADPH 4Na | 137 |
| CoA 3Na | 174 |
| PQQ 2K | 145 |
| cGMP Na | 136 |
| $N^2$-MBcGMP Na | 139 |
| $N^2$-MScGMP Na | 136 |
| $N^2$-MB-8-BrcGMP Na | 135 |
| $N^2$-MB-8-ClPhScGMP Na | 137 |

| Test substance | Number of cells |
|---|---|
| $N^2$-MS-8-BrcGMP Na | 135 |
| $N^2$-MS-8-ClPhScGMP Na | 136 |
| $O^{2'}$-MBcGMP Na | 133 |
| $O^{2'}$-MScGMP Na | 136 |
| $O^{2'}$-MB-8-BrcGMP Na | 135 |
| $O^{2'}$-MB-8-ClPhScGMP Na | 139 |
| $O^{2'}$-MS-8-BrcGMP Na | 138 |
| $N^{2'}$-MS-8-ClPhScGMP Na | 134 |
| 8-BrcGMP Na | 137 |
| 8-ClPhScGMP Na | 139 |
| $N^2$,$O^{2'}$-DBcGMP Na | 141 |
| $N^2$,$O^{2'}$-DScGMP Na | 145 |
| $N^2$,$O^{2'}$-DB-8-BrcGMP Na | 140 |
| $N^2$,$O^{2'}$-DB-8-ClPhScGMP Na | 145 |
| $N^2$,$O^{2'}$-DS-8-BrcGMP Na | 141 |
| $N^2$,$O^{2'}$-DS-8-ClPhScGMP Na | 143 |

It can be seen from Table 2 that all the effective components of the present invention exhibit remarkable growth promotion functions for human fibroblasts. Among these substances, $CoB_{12}$, CoA 3Na and some of the cGMP derivatives exhibit particularly excellent effects.

The fibroblasts present in the corium of the skin are cells which produce constitutive proteins such as collagen and elastin which support the flexibility and elasticity of the skin. Therefore, promotion of the proliferation of these fibroblasts is a necessary condition for promotion of the production of collagen or elastin and, consequently, improvement of the flexibility and elasticity of the skin.

Test Example 2

The action of promoting the production of collagen by the coenzymes and the nucleic acid related substances used in the present invention was evaluated in accordance with the following method:

After hair had been removed from the back of each rat in a group comprising six Wistar 6-week old male rats, a circle of skin was cut off (diameter; about 15 mm) and 0.1 ml of each of 10% ethanol solutions containing 50 mM of the above-described compounds or a 10% ethanol solution containing no compound (control) was repeatedly applied to the skin-free area twice a day for 6 days. Each rat was killed on the seventh day, and then the circular portion was cut out and the weight of granulation (derma) and the amount of hydroxyproline, which is a specific amino acid constituting collagen, were measured as an index of the amount of reproduced collagen. The results are shown in Table 3 in which weights of granulation and amounts of hydroxyproline are shown by relative values obtained on the assumption that those of the control were considered to be 100.

Table 3

| Test substance | Weight of granulation | Amount of hydroxyproline |
|---|---|---|
| No substance added (control) | 100 | 100 |
| $CoB_{12}$ | 181 | 167 |
| FAD 2Na | 128 | 125 |
| NAD | 126 | 124 |
| NADH 2Na | 124 | 121 |
| NADP | 125 | 122 |
| NADPH 4Na | 122 | 120 |
| CoA 3Na | 180 | 182 |
| PQQ 2K | 144 | 120 |
| cGMP Na | 135 | 125 |
| $N^2$-MBcGMP Na | 145 | 128 |
| $N^2$-MScGMP Na | 144 | 127 |
| $N^2$-MB-8-BrcGMP Na | 149 | 130 |
| $N^2$-MB-8-ClPhScGMP Na | 155 | 131 |
| $N^2$-MS-8-BrcGMP Na | 148 | 128 |
| $N^2$-MS-8-ClPhScGMP Na | 153 | 132 |

| Test substance | Weight of granulation | Amount of hydroxyproline |
|---|---|---|
| $O^{2'}$-MBcGMP Na | 138 | 125 |
| $O^{2'}$-MScGMP Na | 139 | 126 |
| $O^{2'}$-MB-8-BrcGMP Na | 145 | 128 |
| $O^{2'}$-MB-8-ClPhScGMP Na | 151 | 130 |
| $O^{2'}$-MS-8-BrcGMP Na | 144 | 128 |
| $O^{2'}$-MS-8-ClPhScGMP Na | 153 | 131 |
| 8-BrcGMP Na | 148 | 130 |
| 8-ClPhScGMP Na | 157 | 133 |
| $N^2,O^{2'}$-DBcGMP Na | 174 | 138 |
| $N^2,O^{2'}$-DScGMP Na | 171 | 138 |
| $N^2,O^{2'}$-DB-8-BrcGMP Na | 172 | 136 |
| $N^2,O^{2'}$-DB-8-ClPhScGMP Na | 174 | 137 |
| $N^2,O^{2'}$-DS-8-BrcGMP Na | 171 | 135 |
| $N^2,O^{2'}$-DS-8-ClPhScGMP Na | 173 | 134 |

As seen from the results shown in Table 3, increases in the weights of regenerated granulation and the amounts of hydroxyproline are apparent in respect of all the active principles of the present invention. This result means that the amount of collagen in the derma increases. Among these substances, CoA, $CoB_{12}$ and some of the cGMP derivatives exhibited particularly excellent effects.

The above results show that, as a result of the promotion of the proliferation of fibroblasts present in the derma of the skin, increase in the weight of dermal granulation and promotion of the production of collagen enable the prevention and remedy of the aging of the skin such as reduction in elasticity and the occurrence of wrinkles.

Test Example 3

The effect of the coenzymes and the nucleic acid related substances against the strength of collagen elastin fibril was conducted as follows:

After hair had been removed from the back of each rat in a group comprising eight Wistar 6-week old male rats, the back of the rat was cut along the median line of the back by a scalpel to make a linear cut portion having a length of 4 cm, and the cut portion was sewed up at 3 positions at equally spaced intervals by Michel's clips. The Michel's clips were removed 3 days after the sewing. 0.1 ml of each of physiological saline containing 50 mM of the above described compound or a physiological saline containing no compound (control) was repeatedly applied to the cut portion once a day for 7 days. Each rat was killed on the eighth day, and the skin of the cut portion was cut out to prepare three samples per rat, each sample being a strip of skin having a width of 1 cm crossing the cutting line.

The tensile strength of the sample up to breakage point was measured by a tensiometer to determine the strength of reproduced collagen elastin fibril. The results obtained are shown in Table 4. In the Table, values of the strength are shown as relative values obtained on the assumption that those of the control were 100.

Table 4

| Test substance | Tensile strength |
|---|---|
| No substance added (control) | 100 |
| $CoB_{12}$ | 122 |
| FAD 2Na | 116 |
| NAD | 115 |
| NADH 2Na | 113 |
| NADP | 113 |
| NADPH 4Na | 111 |
| CoA 3Na | 139 |
| PQQ 2K | 112 |
| cGMP Na | 118 |
| $N^2$-MBcGMP Na | 120 |
| $O^{2'}$-MBcGMP Na | 121 |
| $N^2,O^{2'}$-DBcGMP Na | 123 |
| $N^2,O^{2'}$-DScGMP Na | 123 |
| 8-BrcGMP Na | 121 |

In the above test, a relative value of the strength of more than 110 is considered to show an increase in the tensile strength. Therefore, from the test result shown in Table 4, increases in the tensile strength of collagen elastin fibril are apparent in respect of all the active components of the present invention.

Among these, CoA 3Na, some cGMP derivatives and $CoB_{12}$ show particularly excellent effect. These facts show the possibility of increasing elasticity and flexibility of skin since the tensile strength of collagen elastin fibril is increased.

Examples

Example 1

Polyoxyethylene castor oil, a perfume, and methyl parahydroxybenzoate were dissolved in ethanol and the ethanol solution was added to an aqueous solution in which components other than these compounds had been dissolved so that the compounds became soluble in the aqueous solution. In this way, each of the toilet water samples I to III was prepared. The components used in these toilet water samples are shown in Table 5 in which the numerical values are shown in terms of % by weight (those in the following tables are shown in the same way also).

Table 5

| Component | Agent of this invention | Comparative Agent | |
|---|---|---|---|
| | I | II | III |
| $CoB_{12}$ | 0.05 | - | - |
| Vitamin $B_{12}$ | - | 0.05 | - |
| Glycerin | 3.0 | 3.0 | 3.0 |
| Ethanol | 10.0 | 10.0 | 10.0 |
| 1,3-Buthylene glycol | 4.0 | 4.0 | 4.0 |
| Citric acid | 0.02 | 0.02 | 0.02 |
| Sodium citrate | 0.05 | 0.05 | 0.05 |
| Purified water | 82.18 | 82.18 | 82.23 |
| Polyoxyethylene castor oil | 0.5 | 0.5 | 0.5 |
| Perfume | 0.1 | 0.1 | 0.1 |
| Methyl para-hydroxybenzoate | 0.1 | 0.1 | 0.1 |

The effectiveness of the toilet water samples I to III prepared by the above-described method was evaluated in accordance with the following method:

Two groups each of which comprised 20 statistically-equivalent women (30 to 50 years old) were selected and the toilet water samples I and II were applied separately to the right and left face portions, respectively, of each woman in one group and the toilet water samples I and III were respectively applied to same portions of each woman in the other group by a half face method twice a day (in the morning and at night) for 3 months. The degree of improvement in terms of elasticity, complexion, roughness, and wrinkling of the skin were then examined. The results are shown in Tables 6 and 7.

Table 6

| Evaluation items | I (this invention) is better | I (this invention) is slightly better | Same | III (comparison agent) is slightly better | III (comparison agent) is better |
|---|---|---|---|---|---|
| Improvement in elasticity of the skin | 9 | 7 | 4 | 0 | 0 |
| Improvement in complexion and roughness of the skin | 10 | 5 | 5 | 0 | 0 |
| Improvement in wrinkling | 11 | 5 | 4 | 0 | 0 |

Table 7

| Evaluation items | I (this invention) is better | I (this invention) is slightly better | Same | II (comparison agent) is slightly better | II (comparison agent) is better |
|---|---|---|---|---|---|
| Improvement in elasticity of the skin | 6 | 9 | 5 | 0 | 0 |
| Improvement in complexion and roughness of the skin | 8 | 6 | 6 | 0 | 0 |
| Improvement in wrinkling | 5 | 9 | 6 | 0 | 0 |

As seen from the results shown in Tables 6 and 7, the toilet water I of the present invention containing $CoB_{12}$ exhibited an excellent effect with respect to improvement in terms of elasticity, complexion, roughness, and wrinkling of the skin, as compared with the toilet water II containing vitamin $B_{12}$ - (cyanocobalamine) and the toilet water III to which none of such substances was added.

On the other hand, slight improvements were recognized in the toilet water II to which vitamin $B_{12}$ was added but these effects were greatly inferior to those of the toilet water I.

It is apparent from these results that $CoB_{12}$ has excellent effects in terms of activation of the skin and prevention of aging of the skin.

In addition, during and after the use of the toilet water samples I to III for 3 months, no abnormality was observed in the state of the skin. The results of safety tests conducted for $CoB_{12}$ which was used in the toilet water I of the present invention are shown below.

| | | |
|---|---|---|
| Acute oral toxicity (rat) | : | $LD_{50}$ value; 5 g/kg or more |
| **Skin irritating property** | | |
| (1) Primary skin irritation (guinea pig) | : | 5%; non-irritant |
| (2) Phototoxicity (guinea pig) | : | negative |
| (3) Ocular-mucous membrane irritation (rabbit) | : | 5%, non-irritant |
| **Skin sensitizing property** | | |
| (1) Skin sensitization (guinea pig) | : | negative |
| (2) Photosensitization (guinea pig) | : | negative |
| Human patch test | : | 5%; non-irritant |

It was confirmed by the above-described results that $CoB_{12}$ has a very high level of safety.

## Example 2

The components 1 to 6 and the components 7 to 10 shown in Table 8 were separately dissolved by heating them and they were then mixed and emulsified. The component 11 was added to the emulsion while it was being cooled and uniformly dispersed therein to prepare each of the creams shown in Table 8.

Table 8

| Component | This invention | Comparison |
|---|---|---|
| 1 Lanolin | 2.5 | 2.5 |
| 2 Sorbitan monostearate | 5.0 | 5.0 |
| 3 Polyoxyethylene sorbitan monostearate | 2.0 | 2.0 |
| 4 Beeswax | 10.0 | 10.0 |
| 5 Liquid paraffin | 22.0 | 22.0 |
| 6 Hardened oil | 23.0 | 23.0 |
| 7 Ethyl para-hydroxybenzoate | 0.2 | 0.2 |
| 8 Borax | 0.5 | 0.5 |
| 9 FAD 2Na | 0.5 | - |
| 10 Purified water | 33.8 | 34.3 |
| 11 Perfume | 0.5 | 0.5 |

The effectiveness of the thus-prepared creams was measured in accordance with the following method:

The samples of the cream of the present invention and of the comparison cream were applied separately to the right and left face portions, respectively, of each woman in a group comprising 20 women (30 to 50 years old) twice a day (in the morning and at night) by a half face method for 3 months. The degree of improvement in terms of elasticity, complexion, roughness and wrinkling of the skin was then examined. The results obtained are shown in table 9.

Table 9

| Evaluation items | Cream of this invention is better | Cream of this invention is slightly better | Same | Comparison cream is slightly better | Comparison cream is better |
|---|---|---|---|---|---|
| Improvement in elasticity of the skin | 10 | 4 | 6 | 0 | 0 |
| Improvement in complexion and roughness of the skin | 11 | 4 | 5 | 0 | 0 |
| Improvement in wrinkling | 8 | 6 | 6 | 0 | 0 |

As seen from the results shown in Table 9, the cream of the present invention with which FAD 2Na was mixed exhibits an excellent effect in all of the evaluation items, as compared with the comparison cream to which FAD 2Na was not added. In addition, during and after the use of the cream of the present invention for 3 months, no abnormality was observed in the state of the skin.

When the safety of FAD 2Na was tested in the same way as in Example 1, no problem was observed with respect to skin irritation, etc., and it was thus confirmed that FAD 2Na has a very high level of safety.

Example 3

The components 1 and 2 shown in Table 10 were uniformly mixed and component 7 was added to the mixture under agitation. After components 1 and 2 had been completely dissolved in component 7 at about 60°C, an ethanol (component 3) solution in which components 5 and 6 had been uniformly mixed and dissolved and component 4 were added in turn to the aqueous solution and mixed therein to prepare each of the face packs shown in Table 10.

Table 10

| Component | This invention | Comparison |
|---|---|---|
| 1 Polyvinyl acetate resin | 25.0 | 25.0 |
| 2 Glycerine | 10.0 | 10.0 |
| 3 Ethanol | 10.0 | 10.0 |
| 4 NAD Na | 1.0 | - |
| 5 Methyl para-hydroxybenzoate | 0.1 | 0.1 |
| 6 Perfume | 0.1 | 0.1 |
| 7 Purified water | 53.8 | 54.8 |

The effectiveness of each pack was evaluated in the same way as that of Example 1 except that each of the packs was used on the women's faces twice a week for 6 months. The results are shown in Table 11.

Table 11

| Evaluation items | Pack of this invention is better | Pack of this invention is slightly better | Same | Comparison pack is slightly better | Comparison pack is better |
|---|---|---|---|---|---|
| Improvement in elasticity of the skin | 8 | 6 | 6 | 0 | 0 |
| Improvement in complexion and roughness of the skin | 10 | 5 | 5 | 0 | 0 |
| Improvement in wrinkling | 9 | 7 | 4 | 0 | 0 |

As seen from the results shown in Table 11, the pack of the present invention with which NAD Na was mixed exhibits an excellent effect in all of the evaluation items, as compared with the comparison pack to which NAD Na was not added. In addition, during and after the use of the pack of the present invention for 6 months, no abnormality was observed in the state of the skin.

When the safety of NAD Na was tested in the same way as in Example 1, no problem was observed with respect to skin irritation, etc., and it was thus confirmed that NAD Na has a very high level of safety.

Example 4

The components 1 to 6 and the components 7 to 11 shown in Table 12 were separately mixed and dissolved and a solution containing components 7 to 11 was then added to a solution containing components 1 to 6 under agitation to prepare each of the lotions shown in Table 12.

Table 12

| Component | This invention | Comparison |
|---|---|---|
| 1 Propylene glycol | 4.0 | 4.0 |
| 2 Ethanol | 10.0 | 10.0 |
| 3 Polyoxyethylene castor oil | 1.0 | 1.0 |
| 4 Perfume | 0.2 | 0.2 |
| 5 Dibutylhydroxytoluene | 0.01 | 0.01 |
| 6 Methyl para-hydroxybenzoate | 0.1 | 0.1 |
| 7 NADP Na | 0.1 | - |
| 8 Citric acid | 0.2 | 0.2 |
| 9 Disodium hydrogen phosphate | 0.3 | 0.3 |
| 10 Disodium edetate | 0.05 | 0.05 |
| 11 Purified water | 84.04 | 84.14 |

The effectiveness of each lotion was tested in a similar manner to that of Example 1. The results are shown in Table 13.

Table 13

| Evaluation items | Lotion of this invention is better | Lotion of this invention is slightly better | Same | Comparison lotion is slightly better | Comparison lotion is better |
|---|---|---|---|---|---|
| Improvement in elasticity of the skin | 7 | 8 | 5 | 0 | 0 |
| Improvement in complexion and roughness of the skin | 7 | 10 | 3 | 0 | 0 |
| Improvement in wrinkling | 6 | 8 | 6 | 0 | 0 |

As seen from the results shown in Table 13, the lotion of the present invention with which NADP Na was mixed exhibits an excellent effect in all of the evaluation items, as compared with the comparison pack to which NADP Na was not added. In addition, during and after the use of the lotion of the present invention for 3 months, no abnormality was observed in the state of the skin.

When the safety of NADP Na was tested in the same way as in Example 1, no problem was observed with respect to skin irritation, etc., and it was thus confirmed that NADP Na has a very high level of safety.

Example 5

The components 1 to 3 shown in Table 14 were mixed under agitation and then dissolved by heating at 80°C. Components 4 to 6 and component 8 were added in turn to the obtained solution and uniformly emulsified therein. Component 7 was added to the resulting emulsion and the obtained mixture was then cooled to room temperature to prepare each of the homogeneous skin milks shown in Table 14.

Table 14

| Component | This invention | Comparison |
|---|---|---|
| 1 Liquid paraffin | 20 | 20 |
| 2 Stearyl alcohol | 5 | 5 |
| 3 Isopropyl myristate | 2 | 2 |
| 4 CoA 3Na | 0.3 | - |
| 5 Sodium N-lauroyl glutamate | 2 | 2 |
| 6 Methyl para-hydroxybenzoate | 0.2 | 0.2 |
| 7 Perfume | 0.2 | 0.2 |
| 8 Purified water | 70.3 | 70.6 |

The effectiveness of the thus-obtained skin milks was tested in a similar manner to that of Example 1. The results are shown in Table 15.

Table 15

| Evaluation items | Skin milk of this invention is better | Skin milk of this invention is slightly better | Same | Comparison skin milk is slightly better | Comparison skin milk is better |
|---|---|---|---|---|---|
| Improvement in elasticity of the skin | 9 | 5 | 6 | 0 | 0 |
| Improvement in complexion and roughness of the skin | 10 | 6 | 4 | 0 | 0 |
| Improvement in wrinkling | 7 | 7 | 6 | 0 | 0 |

As seen from the results shown in Table 15, the skin milk of the present invention with which CoA 3Na was mixed exhibits an excellent effect in all of the evaluation items, as compared with the comparison skin milk to which CoA 3Na was not added. In addition, during and after the use of the skin milk of the present invention for 3 months, no abnormality was observed in the state of the skin.

When the safety of CoA 3Na was tested in the same way as in Example 1, no problem was observed with respect to skin irritation, etc., and it was thus confirmed that CoA 3Na has a very high level of safety.

Example 6

The components 1 to 4 shown in Table 16 were dissolved by heating at 80°C. On the other hand, components 5 to 9 were dissolved by heating at 80°C and the obtained solution was then added to the oil phase solution (containing the components 1 to 4) which had been separately produced. The resulting mixture was emulsified and component 10 was then added to the obtained emulsion in the course of cooling to room temperature to prepare each of the milky lotions shown in Table 16.

Table 16

| Component | This invention | Comparison |
|---|---|---|
| 1 Lanolin alcohol | 5.0 | 5.0 |
| 2 Isopropyl myristate | 2.0 | 2.0 |
| 3 Stearic acid | 5.0 | 5.0 |
| 4 Glycerin monostearate | 1.0 | 1.0 |
| 5 Triethanolamine | 0.8 | 0.8 |
| 6 Propylene glycol | 6.0 | 6.0 |
| 7 PQQ 2K | 0.1 | - |
| 8 Methyl para-hydroxybenzoate | 0.3 | 0.3 |
| 9 Purified water | 79.6 | 79.7 |
| 10 Perfume | 0.2 | 0.2 |

The effectiveness of thus-prepared milky lotions was tested in a similar manner to that of Example 1. The results are shown in Table 17.

Table 17

| Evaluation items | Milky lotion of this invention is better | Milky lotion of this invention is slightly better | Same | Comparison milky lotion is slightly better | Comparison milky lotion is better |
|---|---|---|---|---|---|
| Improvement in elasticity of the skin | 9 | 5 | 6 | 0 | 0 |
| Improvement in complexion and roughness of the skin | 7 | 8 | 5 | 0 | 0 |
| Improvement in wrinkling | 11 | 6 | 3 | 0 | 0 |

As seen from the results shown in Table 17, the milky lotion of the present invention with which PQQ 2Na was mixed exhibits an excellent effect in all of the evaluation items, as compared with the comparison milky lotion to which PQQ 2Na was not added. In addition, during and after the use of the milky lotion of the present invention for 3 months, no abnormality was observed in the state of the skin.

When the safety of PQQ 2Na was tested in the same way as in Example 1, no problem was observed with respect to skin irritation, etc., and it was thus confirmed that PQQ 2Na has a very high level of safety.

Example 7

A uniform mixture of the components 3 and 7 to 9 shown in Table 18 was added to an aqueous solution in which other components were dissolved so as to become soluble therein to prepare each of the toilet water samples shown in Table 18.

Table 18

| Component | This invention | Comparison |
|---|---|---|
| 1 $N^2,O^{2'}$-DBcGMP Na | 0.1 | - |
| 2 Glycerin | 3.0 | 3.0 |
| 3 Ethanol | 10.0 | 10.0 |
| 4 Citric acid | 0.02 | 0.02 |
| 5 Sodium citrate | 0.05 | 0.05 |
| 6 Purified water | 86.33 | 86.43 |
| 7 Polyoxyethylene castor oil | 0.5 | 0.5 |
| 8 Perfume | Trace | Trace |
| 9 Preservative | Trace | Trace |

The effectiveness of thus-prepared toilet waters was actually measured by the same method as set forth in Example 1.

The toilet water samples of the present invention and of the comparison were applied separately to the right and left face portions, respectively, of each woman in a group comprising 20 women (30 to 50 years old) by a half face method twice a day (in the morning and at night) for 3 months. The degree of improvement in terms of elasticity, complexion, roughness, and wrinkles of the skin was then examined. The results are shown in Table 19.

Table 19

| Evaluation items | Toilet water of this invention is better | Toilet water of this invention is slightly better | Same | Comparison toilet water is slightly better | Comparison toilet water is better |
|---|---|---|---|---|---|
| Improvement in elasticity of the skin | 11 | 6 | 3 | 0 | 0 |
| Improvement in complexion and roughness of the skin | 8 | 7 | 5 | 0 | 0 |
| Improvement in wrinkling | 10 | 6 | 4 | 0 | 0 |

As seen from the results shown in Table 19, the toilet water of the present invention containing $N^2,O^{2'}$-DBcGMP Na exhibited an excellent effect with respect to improvement in terms of elasticity, complexion, roughness, and wrinkling of the skin, as compared with the toilet water to which $N^2,O^{2'}$-DBcGMP Na was not added.

It is apparent from these results that $N^2,O^{2'}$-DBcGMP Na has an excellent effect in terms of activation of the skin and prevention of aging of the skin.

In addition, during and after the use of the toilet water samples I to III for 3 months, no abnormality was observed in the state of the skin. The safety tests for $N^2,O^{2'}$-DBcGMP Na were conducted by the same method as set forth in Example 1, and problems such as skin irritating property were not found.

It was not confirmed by the above-described results that $N^2,O^{2'}$-BDcGMP has a very high level of safety.

Example 8

The components 1 to 5 and the components 6 to 10 shown in Table 20 were separately dissolved by heating at 80°C and the two solutions were mixed and emulsified. Component 11 was then added to the obtained emulsion under cooling and uniformly dispersed therein to prepare each of the milky lotions shown in Table 20.

Table 20

| Component | This invention | Comparison |
|---|---|---|
| 1 Stearic acid | 2.5 | 2.5 |
| 2 Cetanol | 1.5 | 1.5 |
| 3 Petrolatum | 5.0 | 5.0 |
| 4 Liquid paraffin | 10.0 | 10.0 |
| 5 Polyethylene glycol mono-oleate | 2.0 | 2.0 |
| 6 Polyethylene glycol 1500 | 3.0 | 3.0 |
| 7 Triethanolamine | 1.0 | 1.0 |
| 8 cGMP Na | 1.0 | - |
| 9 Preservative | Trace | Trace |
| 10 Purified water | 74.0 | 75.0 |
| 11 Perfume | Trace | Trace |

The effectiveness of the thus-prepared milky lotions was examined by allowing 20 women to use them in a similar way to that of Example 1. The results are shown in Table 21.

Table 21

| Evaluation items | Milky lotion of this invention is better | Milky lotion of this invention is slightly better | Same | Comparison milky lotion is slightly better | Comparison milky lotion is better |
|---|---|---|---|---|---|
| Improvement in elasticity of the skin | 10 | 6 | 4 | 0 | 0 |
| Improvement in complexion and roughness of the skin | 7 | 7 | 6 | 0 | 0 |
| Improvement in wrinkling | 7 | 6 | 7 | 0 | 0 |

As seen from the results shown in Table 21, the milky lotion of the present invention with which cGMP Na was mixed exhibited an excellent effect with respect to all of the evaluation items, as compared with the milky lotion in which cGMP Na was not mixed. In addition, during and after the use of the milky lotion of the present invention for 3 months, no abnormality was observed in the state of the skin.

When the safety of cGMP Na was tested by the same method as that of Example 1, it was confirmed that cGMP Na had no problem with respect to skin irritation, etc., and had an extremely high level of safety.

Example 9

When the effect of preventing the aging of the skin was examined in a similar way to that of Example 7 except that $N^2$-MBcGMP Na, $O^{2'}$-MBcGMP Na, $N^2$,$O^{2'}$-DScGMP Na, or 8-BrcGMP Na was used in place of $N^2$,$O^{2'}$-DBcGMP Na, excellent effects similar to those of Example 7 were obtained.

Example 10

The effectiveness of cGMP which is an active principle of the present invention and its derivatives was tested in accordance with the following method:

After hair had been removed from the back of each colored guinea pig (3 guinea pigs in a group), a cream with which 0.5% of an active principle was mixed (cGMP or its derivatives in place of FAD 2Na used in Example 2) and a cream containing no active principle were respectively applied to the back of each guinea pig twice a day (in the morning and at night) every day for 14 days. The difference in colour tone of the skin before and after the application was measured by a color-difference meter. The results are shown in Table 22.

Table 22

| Test substance | Before application | After application |
|---|---|---|
| No substance was added (control) | 36.2 | 36.3 |
| cGMP Na | 36.0 | 43.0 |
| $N^2$-MBcGMP Na | 36.2 | 44.6 |
| $N^2$-MScGMP Na | 36.1 | 44.6 |
| $O^{2'}$-MBcGMP Na | 36.3 | 43.7 |
| $O^{2'}$-MScGMP Na | 36.3 | 43.8 |
| $N^2,O^{2'}$-DBcGMP Na | 36.2 | 45.5 |
| $N^2,O^{2'}$-DScGMP Na | 36.1 | 45.3 |
| 8-BrcGMP Na | 36.2 | 45.4 |
| 8-ClPhScGMP Na | 36.2 | 45.3 |

The numerical values shown in the table are L-values (lightness) and a larger value indicates a whiter skin.

As seen from the results shown in Table 22, the cream with which cGMP Na or its derivatives was mixed has an excellent decoloration effect as compared with the control.

Example 11

Creams having the compositions shown in Table 23 were prepared and then applied to a discolored spot or freckled portion of the skin of each of 6 men and women in the morning and at night twice a day for 3 months. The lightness of the discolored spot portions was measured by a color-difference meter before and after the application. The results are shown in Table 24.

Table 23

| Component | Cream of this invention | | Comparison cream |
|---|---|---|---|
| | I | II | |
| White petrolatum | 25.0 | 25.0 | 25.0 |
| Stearyl alcohol | 25.0 | 25.0 | 25.0 |
| Propylene glycol | 12.0 | 12.0 | 12.0 |
| Sodium lauryl sulfate | 1.0 | 1.0 | 1.0 |
| $N^2,O^{2'}$-DBcGMP Na | 0.1 | - | - |
| cGMP Na | - | 0.1 | - |
| Preservative | Trace | Trace | Trace |
| Purified water | 36.9 | 36.9 | 37.0 |

Table 24

| | Cream of this invention | | Comparison cream |
|---|---|---|---|
| | I | II | |
| Before application | 44.7 | 44.6 | 44.7 |
| After application | 50.5 | 49.0 | 44.8 |

The numerical values obtained before and after the application shown in the table are L-values (lightness).

As seen from the results shown in Table 24, the cream with which $N^2,O^{2'}$-DBcGMP Na or cGMP Na was mixed exhibits an increased lightness of the pigment spots, as compared with the comparison cream with which none of these substances was mixed. In addition, during and after the use of the creams for 3 months, no abnormality was observed in the state of the skin.

It is also clear from the above results that cGMP and derivatives thereof have the effect of suppressing discolored spots and freckles in addition to the effect of preventing aging of the skin.

Example 12

Creams were prepared by the same method as set forth in Example 11, except that $N^2$-MBcGMP Na, $O^{2'}$-MBcGMP Na or $\epsilon$-BrcGMP Na was used instead of $N^2,O^{2'}$-DBcGMP Na and cGMP Na. The properties of the creams were determined by the same method as set forth in Example 11 and the results similar to those in Example 11 were obtained.

**Claims**

1. A cosmetic agent for application to skin, which comprises at least one compound selected from the group consisting of 5'-deoxyadenosylcobalamine or salts thereof, flavin adenine dinucleotide or salts thereof; β-nicotinamide adenine dinucleotide, a reduced form thereof, or their salts; β-nicotinamide adenine dinucleotide phosphate, a reduced form thereof, or their salts; coenzyme A or salts thereof; pyrroloquinoline quinone or salts thereof; and compounds having a fundamental skeleton given by the following formula:

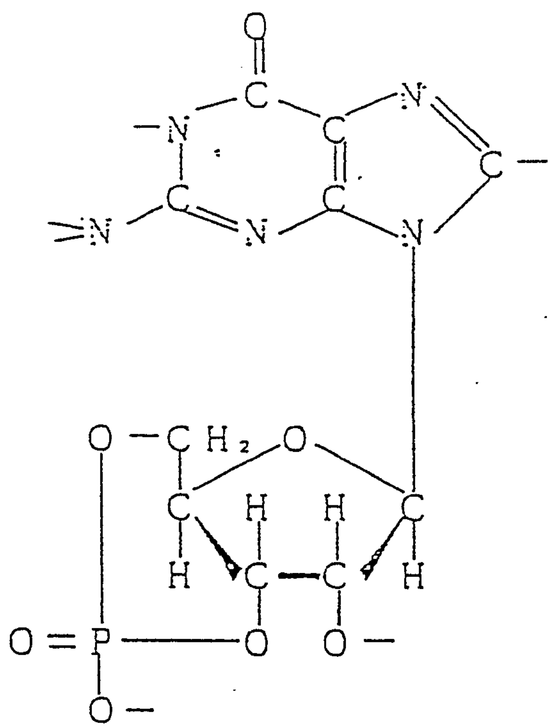

··· [ I ]

2. A cosmetic agent as set forth in claim 1 wherein the compound is 5'-deoxyadenosylcobalamine or a salt thereof.

3. A cosmetic agent as set forth in claim 1 wherein the compound is flavin adenine dinucleotide or a salt thereof.

4. A cosmetic agent as set forth in claim 1 wherein the compound is selected from the group consisting of β-nicotinamide adenine dinucleotide, β-nitocinamide adenine dinucleotide phosphate, reduced form thereof, and their salts.

5. A cosmetic agent as set forth in claim 1 wherein the compound is coenzyme A or a salt thereof.

6. A cosmetic agent as set forth in claim 1 wherein the compound is pyrrolo-quinoline quinone or a salt thereof.

7. A cosmetic agent as set forth in claim 1 wherein the compound having the skeleton given by the formula [I] has the following formula [II]:

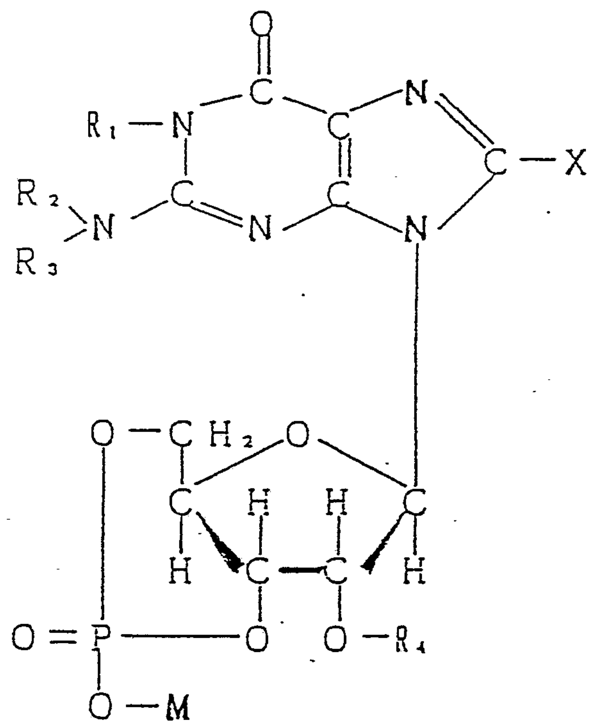

... [ II ]

wherein $R_1$, $R_2$, $R_3$, and $R_4$ are each a hydrogen atom, an acyl group having 1 to 22 carbon atoms, or an alkyl group having 1 to 22 carbon atoms; X is a hydrogen atom, a halogen atom, a mercapto group, a thio-containing group, an amino group, an aminoalkyl group, or a hydroxyl group; and M is a hydrogen atom or a salt-forming cation.

8. A cosmetic agent as set forth in claim 1 wherein an amount of the compound is 0.001 to 5% by weight.

9. A cosmetic agent as set forth in claim 1 wherein an amount of the compound is 0.01 to 2% by weight.

10. A cosmetic agent as set forth in claim 1 wherein the cosmetic agent comprises 0.001 to 5% by weight of the compound, 0.5 to 12% by weight of a surfactant, 2 to 15% by weight of a humectant, 0 to 80% by weight of an oil, very small amount of preservative and 11 to 95% by weight of purified water.